Europäisches Patentamt

European Patent Office

Office européen des brevets

(11) Numéro de publication: **0 338 618 B1**

(12) **FASCICULE DE BREVET EUROPEEN**

(45) Date de publication de fascicule du brevet: **25.01.95**　(51) Int. Cl.6: **A61B 8/06**

(21) Numéro de dépôt: **89200927.5**

(22) Date de dépôt: **13.04.89**

Le dossier contient des informations techniques présentées postérieurement au dépôt de la demande et ne figurant pas dans le présent fascicule.

(54) **Dispositif de mesure par corrélation de la vitesse d'organes en mouvement et d'écoulements sanguins.**

(30) Priorité: **19.04.88 FR 8805146**

(43) Date de publication de la demande:
**25.10.89 Bulletin 89/43**

(45) Mention de la délivrance du brevet:
**25.01.95 Bulletin 95/04**

(84) Etats contractants désignés:
**DE FR GB**

(56) Documents cités:
**FR-A- 1 522 965**
**FR-A- 2 447 041**
**FR-A- 2 590 790**
**US-A- 4 324 258**

**MEDICAL & BIOLOGICAL ENGINEERING & COMPUTING, vol. 21, no. 3, mai 1983, pages 358-364,IFMBE, Stevenage, Herts, GB; D. CA-THIGNOL et al.: "Description et application d'un vélocimètre ultrasonore doppler pour les petits vaisseaux"**

(73) Titulaire: **LABORATOIRES D'ELECTRONIOUE PHILIPS**
**3, Avenue Descartes**
**F-94450 Limeil-Brévannes (FR)**
(84) Etats contractants désignés:
**FR**

(73) Titulaire: **Philips Electronics N.V.**
**Groenewoudseweg 1**
**NL-5621 BA Eindhoven (NL)**
(84) Etats contractants désignés:
**DE GB**

(72) Inventeur: **Bonnefous, Odile**
**Société Civile S.P.I.D.**
**209 Rue de l'Université**
**F-75007 Paris (FR)**

(74) Mandataire: **Charpail, François et al**
**Société Civile S.P.I.D.**
**156, Boulevard Haussmann**
**F-75008 Paris (FR)**

**Description**

La présente invention concerne un dispositif de mesure de la vitesse d'organes en mouvement et d'écoulements sanguins, comportant, d'une part, un circuit d'intercorrélation délivrant à partir de deux lignes échographiques successives des valeurs de fonctions de corrélation, et, d'autre part, un circuit de multiplexage-interpolation fournissant à partir desdites valeurs des fonctions de corrélation une estimée de la vitesse et la valeur du pic de corrélation correspondant.

L'invention trouve une application particulièrement avantageuse dans le domaine de l'exploration échographique d'organes en mouvement, tels que les parois du coeur, et d'écoulements sanguins dans les vaisseaux.

Le problème technique général à résoudre par tout dispositif de mesure de la vitesse d'organes en mouvement et d'écoulements sanguins est d'obtenir une estimation aussi exacte que possible de la vitesse axiale du mouvement étudié afin de réaliser, à l'aide de dispositifs de visualisation, des images précises des organes et des écoulements sanguins soumis à une exploration échographique ultrasonore.

Depuis plusieurs années, diverses solutions à ce problème technique ont été proposées. En ce sens, la demande de brevet français FR-A-2 590 790 décrit un dispositif de mesure de la vitesse d'organes en mouvement et d'écoulements sanguins conforme au préambule qui utilise le fait que les signaux ultrasonores successifs rétrodiffusés par une cible en mouvement, lorsque l'émission est récurrente à la période de récurrence T, sont reliés par l'équation suivante :

$$S_{n+1}(t) = S_n(t-\tau)$$

Ceci signifie que le signal $n+1$ est la réplique du signal n précédent à un décalage temporel $\tau$ près. Ce dernier représente le temps supplémentaire nécessaire à l'onde ultrasonore pour parcourir le trajet transducteur-cible-transducteur, d'un tir à l'autre. Autrement dit:

$$\tau = 2VT/C$$

où V est la vitesse de la cible et C la vitesse du son. On voit qu'une mesure de $\tau$ permet de mesurer la vitesse V cherchée.

La fonction d'intercorrélation entre $S_n(t)$ et $S_{n+1}(t)$ définie par :

$$C_{n,n+1}(to,u) = \int_{to}^{to+W} S_{n+1}(t+u)S_n(t)dt$$

vérifie

$$C_{n,n+1}(to+u) = C_{nn}(to,u-\tau)$$

Le temps to est lié à la profondeur d'exploration z par $to = 2z/C$.

La fonction $C_{nn}(to,u)$ est une fonction d'autocorrélation, et, de ce fait, est maximale pour $u = uo$. Ainsi, une mesure du décalage temporel $\tau$, et donc de la vitesse V, peut se faire en cherchant pour quel paramètre u la fonction $C_{n,n+1}(to,u)$ est maximale. A cet effet, la fonction d'intercorrélation est échantillonné, à un pas d'échantillonnage $\Delta t$, entre $u_{min} = -I\Delta t$ et $u_{max} = I\Delta t$ par pas de 1 de façon à obtenir $2I+1$ valeurs de fonctions de corrélation. La valeur maximum de ces $2I+1$ valeurs correspondant à $u = uo$ permet de mesurer $\tau$ en utilisant l'égalité $\tau = uo$.

Afin de s'affranchir des erreurs inhérentes à l'échantillonnage dans la détermination du maximum de la fonction de corrélation, il est possible d'utiliser un circuit de multiplexage-interpolation fournissant à partir des valeurs des fonctions de corrélation une estimée plus précise de la vitesse et la valeur du pic de corrélation correspondant. La demande de brevet français FR-A-2 590 790 donne un exemple de ce type de traitement du signal échographique dans lequel la corrélation entre signaux est une corrélation dite "1 bit" en ce sens que les signaux $S_{n+1}$ et $S_n$ utilisés précédemment sont réduits au signe du signal ultrasonore. On sait que dans ce cas, le pic de la fonction de corrélation est de forme triangulaire isocèle. La connaissance de cette forme permet à partir du point le plus haut et de ses deux voisins de reconstituer complètement, par interpolation linéaire, le pic de corrélation et donc de déterminer avec précision l'emplacement de uo.

Cette méthode connue de mesure des vitesses, reposant sur l'analyse du décalage temporel, présente de sérieux avantages sur d'autres méthodes fondées, par exemple, sur le décalage de fréquence ou de phase. En particulier, elle permet d'utiliser des signaux d'émission à bande large, produisant une bonne résolution axiale de la mesure. De même, compte tenu du fait que la méthode de mesure par corrélation ne subit pas de phénomène de repliement, il est possible de mesurer des vitesses au-delà du seuil généralement imposé par les instruments usuels.

Toutefois, la méthode exposée ci-dessus présente l'inconvénient de rendre possible une erreur, liée à l'échantillonnage, dans la détermination de la position du pic de corrélation. En effet, il peut se produire que le point le plus haut de la fonction de corrélation échantillonnée n'appartient pas au pic de corrélation recherché. Cette situation peut survenir lorsqu'on mesure des flux complexes, comportant des gradients de vitesse importants qui tendent à faire baisser le pic de corrélation. Cette erreur se traduit par des discontinuités brutales dans la reconstitution du profil de la vitesse $V$ en fonction de la profondeur $z$ d'exploration.

Aussi, le problème technique à résoudre par l'objet de la présente demande est de réaliser un dispositif de mesure de la vitesse d'organes en mouvement et d'écoulements sanguins, comportant, d'une part, un circuit d'intercorrélation fonctionnant avec un pas d'échantillonnage $\Delta t$, et délivrant, à partir de deux lignes échographiques successives décalées de $k\Delta t$ ($k = -l, -l+1,...,l$), $2l+1$ valeurs de fonctions de corrélation, et, d'autre part, un circuit de multiplexage-interpolation fournissant à partir desdites valeurs des fonctions de corrélation, une estimée de ladite vitesse et la valeur du pic de corrélation correspondant, dispositif grâce auquel on pourrait déterminer sans ambiguïté la position du maximum du pic de corrélation en éliminant les artéfacts dus aux forts gradients de vitesse.

La solution à ce problème technique consiste, selon la présente invention en ce que lesdits circuits d'intercorrélation et de multiplexage-interpolation fournissent ladite estimée de vitesse et ladite valeur du pic de corrélation à l'intérieur de chacune de N zones définies dans l'intervalle des décalages temporels $[-l\Delta t, +l\Delta t]$, et en ce qu'il comporte également un détecteur de passage par zéro qui définit M segments limités par les valeurs des profondeurs d'exploration pour lesquelles l'estimée de la vitesse passe par zéro dans l'ensemble des N zones, tandis qu'une mémoire enregistre les valeurs cumulées $P_{ij}$ du pic de corrélation correspondant à la ième zone ($i = 1,...,N$) et au jème segment ($j = 1,...,M$), et qu'une unité de détection reconstitue le profil de vitesse cherché en retenant pour chaque segment $j$ le profil de vitesse dans la zone $i$ pour laquelle $P_{ij}$ est maximum.

Ainsi, l'invention consiste essentiellement à "suivre" le pic de corrélation à travers tous les chemins possibles représentés par les N zones et les M segments, le critère de choix étant la valeur cumulée du pic de corrélation sur tous les dessins. Le chemin retenu est celui qui correspond à la plus grande valeur.

La description qui va suivre en regard des dessins annexés, donnés à titre d'exemples non limitatifs, fera bien comprendre en quoi consiste l'invention et comment elle peut être réalisée.

La figure 1 est un diagramme du dispositif de mesure selon l'invention.

La figure 2 est une courbe donnant la fonction de corrélation en fonction de l'échantillonnage dans le cas d'une corrélation 1 bit, ainsi que la structure en zones.

La figure 3 donne, pour chaque zone, les variations de la vitesse estimée en fonction de la profondeur d'exploration, ainsi que la structure en segments.

La figure 4 montre un résultat expérimental obtenu par la Demanderesse.

La figure 5 donne la structure de la mémoire correspondant au résultat de la figure 4.

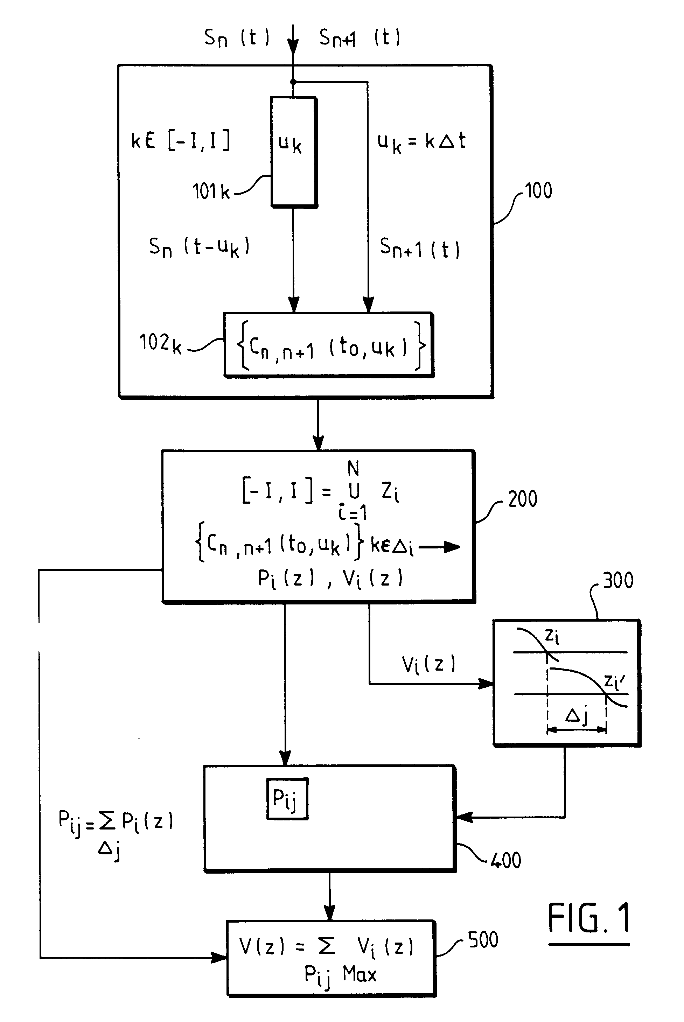

La figure 1 montre, sous forme schématique, un dispositif de mesure de la vitesse d'organes en mouvement et d'écoulements sanguins. Ce dispositif fait partie d'un appareil d'exploration par échographie ultrasonore qui comprend en outre, et non représentés sur la figure 1, au moins un transducteur ultrasonore associé à un étage d'émission périodique d'un signal impulsionnel de fréquence de récurrence déterminée $F = 1/T$ et à un étage de réception des signaux échographiques renvoyés vers le transducteur et de traitement des signaux reçus. La demande de brevet français FR-A-2 590 790 donne une description très détaillée de ces différents étages. L'étage de réception et de traitement comporte, notamment, une ligne à retard d'une période de récurrence $T$ qui permet de recevoir en même temps deux signaux échographiques consécutifs $S_n(t)$ et $S_{n+1}(t)$. Comme on peut le voir à la figure 1, ces deux signaux $S_n$ et $S_{n+1}$ sont traités par un circuit d'intercorrélation 100 fonctionnant avec un pas d'échantillonnage $\Delta t$.

Des lignes à retard $101_k$ décalent le signal $S_n(t)$ de la quantité $u_k = k\Delta t$, le nombre entier $k$ prenant les valeurs $-l, -l+1,...,-1,0,1,...,l-1,l$. Puis, des corrélateurs 102k au nombre de $2l+1$ fournissent $2l+1$ valeurs de fonctions de corrélation, soit :

$$\{C_{n,n+1}(to,uk)\}_{k\in[-l,l]}$$

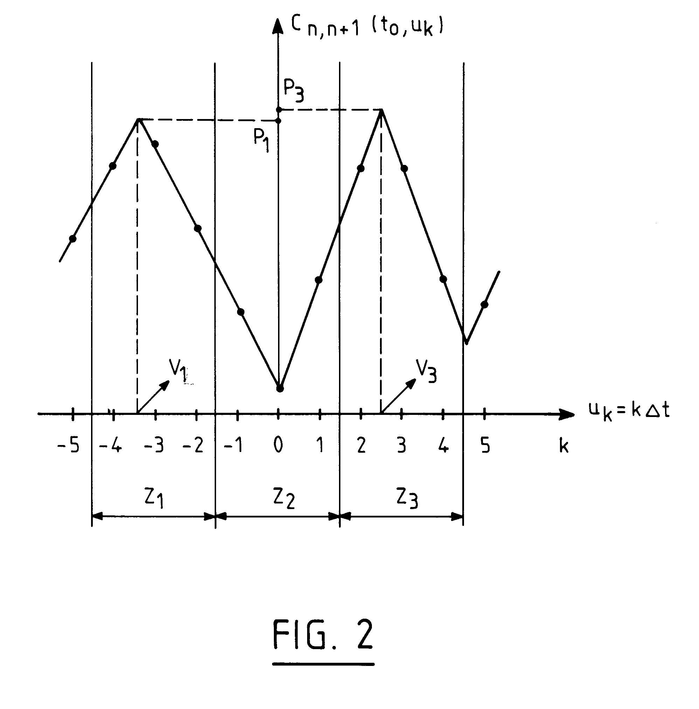

Les corrélateurs 102k peuvent être, par exemple, des corrélateurs "1bit". Et, dans ce cas, la figure 2 montre l'allure de la fonction de corrélation qui, comme on peut le voir, est constituée de pics de corrélation ayant la forme de triangles isocèles. La courbe de la figure 2 correspond à une valeur du nombre $l$ égale à 5, ce qui représente 11 points de corrélation. Cette situation se présente, par exemple, pour une fréquence d'émission de 5MHz et une fréquence d'échantillonnage de 20MHz.

L'ensemble des $2l+1$ valeurs de fonctions de corrélation issues du circuit d'intercorrélation 100 parviennent ensuite à un circuit de multiplexage-interpolation 200. Ce circuit 200 réalise une partition des points de corrélation en les groupant dans N zones $Z_i$ ($i=1,...,N$). La figure 2 montre une telle partition en 3 zones $Z_1$, $Z_2$ et $Z_3$ comprenant chacune 3 points de corrélation. A l'intérieur de chaque zone $Z_i$, le circuit 200 fournit une valeur de l'estimée de la vitesse $V_i$ et du pic de corrélation $P_i$. Ces valeurs étant fonction du temps to, donc de la profondeur d'exploration, elles seront notées $V_i(z)$ et $P_i(z)$. Dans le cas de la corrélation 1 bit (figure 2), le calcul de $V_i(z)$ et de $P_i(z)$ peut se faire par interpolation linéaire construite sur le point le plus haut de chaque zone et des 2 voisins, le sommet du triangle isocèle ainsi reconstruit correspondant au couple $V_i(z)$, $P_i(z)$. Cette méthode d'interpolation est décrite dans la demonade de brevet français FR-A-2 590 790.

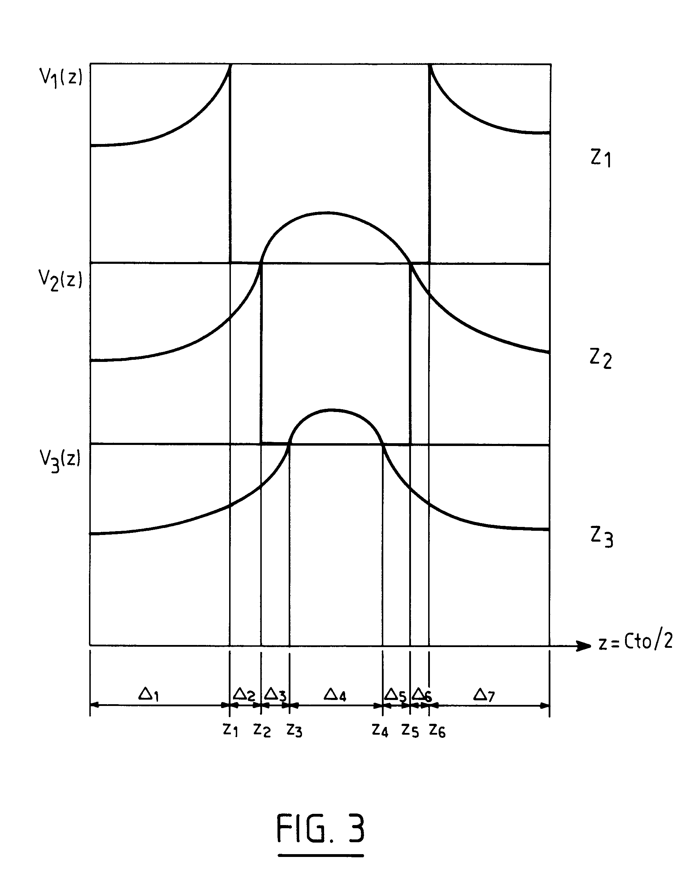

Les N profils de vitesses $V_i(z)$ peuvent être portés graphiquement en fonction z, comme le montre la figure 3. On constate sur cette figure que les profils $V_i(z)$ subissent des passages par 0 correspondant à l'absence de pic de corrélation dans la zone $Z_i$ considérée, c'est le cas par exemple, pour la zone $Z_2$ sur la figure 2. Ces passages par zéro $Z_1$, $Z_2$,...,$Z_j$,...sont détectés par un détecteur de passage par zéro 300 et permettent de définir M segments $\Delta_j$(j-1,...,M) le long de l'axe z. Dans l'exemple de la figure 3, le nombre de segments est $M=7$. Les M segments $\Delta_j$ étant ainsi définis, il est possible à l'intérieur de chacun de ces segments de cumuler pour chaque zone $Z_i$ les valeurs $P_i(z)$ du pic de corrélation de façon à définir un nombre $P_{ij}$ qui peut s'écrire:

$$P_{ij} = \sum_{\Delta_j} P_i(z)$$

Les NxM valeurs des $P_{ij}$ sont rangées dans une mémoire 400 que vient lire une unité de détection 500 de la façon suivante : pour chaque segment $\Delta_j$, on cherche pour quelle zone $Z_i$ le paramètre $P_{ij}$ est maximum. Alors, la portion du profil V(z) total cherché, à l'intérieur du segment $\Delta j$, est précisément $V_i(z)$. Les $V_i(z)$ sont stockés dans une mémoire, la reconstitution s'effectue en allant rechercher ces valeurs lorsque $P_{ij}$ est maximum. Ceci peut se traduire par la relation :

$$V(z) = \sum_{P_{ijMax}} V_i(z)$$

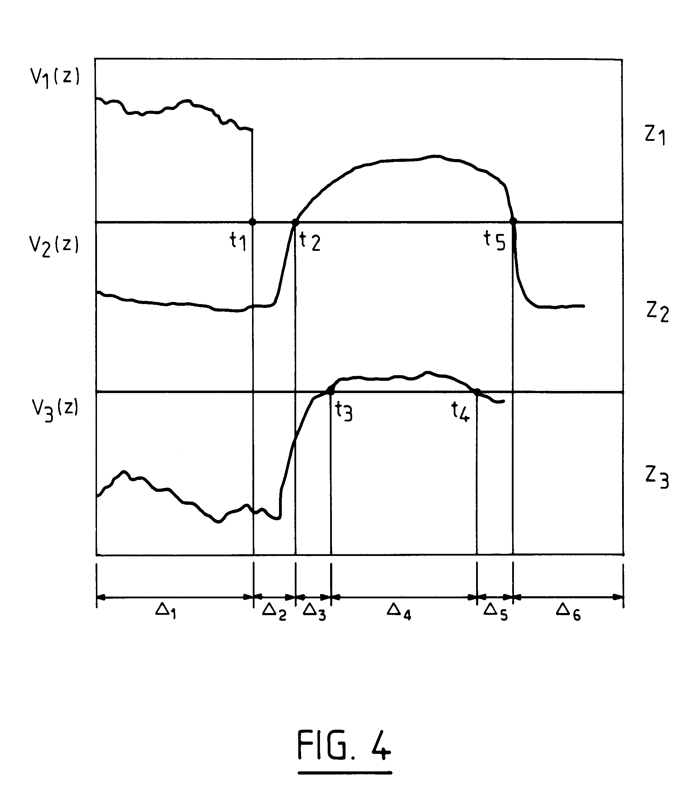

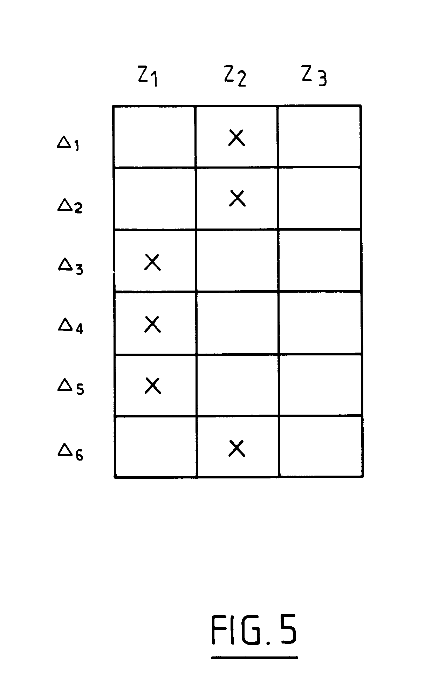

Dans l'exemple expérimentalement obtenu par la Demanderesse et montré à la figure 4, la mémoire 400 avait la structure donnée à la figure 5. Elle comportait $3x6=18$ valeurs cumulées de pics de corrélation. Les valeurs maximales pour chaque segment $\Delta_j$ sont marquées d'une croix. Le profil V(z) cherché est donc donné par : $V_2(z)$ pour les segments $\Delta_1$, $\Delta_2$ puis par $V_1(z)$ pour $\Delta_3$, $\Delta_4$, $\Delta_5$ et, enfin, à nouveau par $V_2(z)$ pour $\Delta_6$.

**Revendications**

**1.** Dispositif de mesure de la vitesse d'organes en mouvement et d'écoulements sanguins, comportant, d'une part, un circuit d'intercorrélation (100) fonctionnant avec un pas d'échantillonnage $\Delta t$, et délivrant, à partir de deux lignes échographiques successives décalées de $k\Delta t$ ($k=-l,-l+1,...,l$), $2l+1$ valeurs de fonctions de corrélation, et, d'autre part, un circuit de multiplexage-interpolation (200) fournissant à partir desdites valeurs des fonctions de corrélation, une estimée de ladite vitesse et la valeur du pic de corrélation correspondant, caractérisé en ce que lesdits circuits d'intercorrélation (100) et de multiplexage-interpolation (200) fournissent ladite estimée de vitesse et ladite valeur du pic de corrélation à l'intérieur de chacune de N zones définies dans l'intervalle des décalages temporels [-l$\Delta t$, +l$\Delta t$], et en ce qu'il comporte également un détecteur de passage par zéro (300) qui définit M segments limités par les valeurs des profondeurs d'exploration pour lesquelles l'estimée de la vitesse passe par zéro dans l'ensemble des N zones, tandis qu'une mémoire (400) enregistre les valeurs cumulées $P_{ij}$ du pic de corrélation correspondant à la ième zone ($i=1,...,N$) et au jème segment ($j=1,...,M$), et qu'une unité de détection (500) reconstitue le profil de vitesse cherché en retenant pour chaque segment j le profil de vitesse dans la zone i pour laquelle $p_{ij}$ est maximum.

4

**2.** Dispositif selon la revendication 1, caractérisé en ce que ledit circuit d'intercorrélation (100) comporte des corrélateurs 1 bit et en ce que ledit circuit de multiplexage-interpolation (200) effectue une interpolation linéaire.

**Claims**

**1.** A device for measuring the speed of moving organs and blood flows, comprising an intercorrelation circuit (100) which operates with a sampling step $\Delta t$ and which supplies, on the basis of two successive echographic lines shifted by $k\Delta t$ ($k$ = -I, -I+1, .., I), 2I+1 correlation function values, and a multiplex/interpolation circuit (200) which supplies, on the basis of said correlation function values, an estimate of said speed and the value of the corresponding correlation peak, characterized in that said intercorrelation circuit (100) and multiplex/interpolation circuit (200) supply said estimate of the speed and said value of the correlation peak within each of N zones defined in the time shift interval [-I$\Delta t$, +$\Delta t$], in that it also comprises a zero-crossing detector (300) which defines M segments which are limited by the values of the scanning depth for which the estimate of the speed passes through zero in the set of N zones, a memory (400) storing the accumulated values $P_{ij}$ of the correlation peak corresponding to the $i^{th}$ zone ($i$ = 1, ..., N) and to the $j^{th}$ segment ($j$ = 1, ..., M), and in that a detector unit (500) reconstructs the speed profile searched by retaining for each segment j the speed profile in the zone i for which $P_{ij}$ is maximum.

**2.** A device as claimed in Claim 1, characterized in that said intercorrelation circuit (100) comprises 1-bit correlators and in that said multiplex/interpolation circuit (200) performs a linear interpolation.

**Patentansprüche**

**1.** Vorrichtung zur Messung der Geschwindigkeit von Organbewegungen und Blutströmungen, die erstens eine Korrelationsschaltung (100) umfaßt, die mit einem Abtastschritt von $\Delta t$ arbeitet und auf der Basis zweier aufeinanderfolgender, um $k\Delta t$ ($k$ = -I, -I+1, ..., I) verschobener Echographielinien 2I+1 Korrelationsfunktionswerte liefert, und zweitens eine Multiplexinterpolationsschaltung (200) umfaßt, die auf der Basis der genannten Korrelationsfunktionswerte eine Schätzung der genannten Geschwindigkeit und des Wertes des entsprechenden Korrelations-Peaks liefert, <u>dadurch gekennzeichnet</u>, daß die genannte Korrelationsschaltung (100) und die Multiplexinterpolationsschaltung (200) die genannte Schätzung der Geschwindigkeit und des Wertes des Korrelations-Peaks innerhalb jeder der N im Intervall der zeitlichen Verschiebungen [-I$\Delta t$, +I$\Delta t$] definierten Zonen liefern und daß außerdem ein Nullstellendetektor (300) vorhanden ist, der M Segmente definiert, die durch die Werte der Untersuchungstiefen begrenzt werden, bei denen der Schätzwert für die Geschwindigkeit in der Gesamtheit der N Zonen eine Nullstelle hat, wobei ein Speicher (400) die kumulierten Werte $P_{ij}$ des der i-ten Zone ($i$ = 1, ..., N) und dem j-tn Segment ($j$ = 1, ... N) entsprechenden Korrelations-Peaks speichert, und wobei eine Detektionseinheit (500) das gesuchte Geschwindigkeitsprofil bildet, indem für jedes Segment j das Geschwindigkeitsprofil in der Zone i, bei der $P_{ij}$ maximal ist, registriert wird.

**2.** Vorrichtung nach Anspruch 1, <u>dadurch gekennzeichnet</u>, daß die genannte Korrelationsschaltung (100) 1-Bit-Korrelatoren umfaßt und daß die genannte Multiplexinterpolationsschaltung (200) eine lineare Interpolation vornimmt.

$S_n(t)$     $S_{n+1}(t)$

$k \in [-I, I]$      $u_k$      $u_k = k\triangle t$

101k

100

$S_n(t - u_k)$      $S_{n+1}(t)$

102k    $\left\{ C_{n,n+1}(t_0, u_k) \right\}$

$$[-I, I] = \bigcup_{i=1}^{N} Z_i$$

200

$$\left\{ C_{n,n+1}(t_0, u_k) \right\} k \in \triangle_i \longrightarrow$$

$$P_i(z), V_i(z)$$

300

$V_i(z)$

$z_i$

$z_{i'}$

$\triangle_j$

$P_{ij}$

$$P_{ij} = \sum_{\triangle_j} P_i(z)$$

400

$$V(z) = \sum V_i(z)$$

$$P_{ij} \text{ Max}$$

500

FIG.1

FIG. 2

FIG. 3

8

FIG. 4

|  | $Z_1$ | $Z_2$ | $Z_3$ |
|---|:---:|:---:|:---:|
| $\triangle_1$ |  | X |  |
| $\triangle_2$ |  | X |  |
| $\triangle_3$ | X |  |  |
| $\triangle_4$ | X |  |  |
| $\triangle_5$ | X |  |  |
| $\triangle_6$ |  | X |  |

## FIG. 5